# EUROPEAN PATENT SPECIFICATION

(11) **EP 1 309 323 B1**
(45) Date of publication and mention of the grant of the patent: **15.11.2006**
(21) Application number: 01962132.5
(22) Date of filing: 13.08.2001
(51) Int. Cl.: A61K 31/215, A61K 31/165, A61K 31/185, A61P 9/00, A61P 35/00, A61P 27/02

(54) **TREATMENT OF ANGIOGENESIS-RELATED DISORDERS USING BENZOYL PHENYLACETIC ACID DERIVATIVES**
BEHANDLUNG VON ANGIOGENESEBEZOGENEN KRANKHEITEN MIT BENZOYL PHENYLESSIGSÄURE DERIVATEN
TRAITEMENT DES TROUBLES RELATIFS À L'ANGIOGENESE À L'AIDE DE DÉRIVÉS DE L'ACIDE BENZOYL PHENYLACETIQUE

(30) Priority: 14.08.2000 US 225133 P
(43) Date of publication of application: 14.05.2003
(73) Proprietor: Alcon, Inc., 6331 Hünenberg (CH)
(72) Inventor: KAPIN, Michael, A., Arlington, TX 76016 (US); BINGAMAN, David, P., Lipan, TX 76462 (US); GAMACHE, Daniel, A., Arlington, TX 76017 (US); GRAFF, Gustav, Cleburne, TX 76031 (US); YANNI, John, M., Burleson, TX 76028 (US)
(74) Representative: Hanna, Peter William Derek
(86) International application number: PCT/US2001/025318
(87) International publication number: WO 2002/013804

(56) References cited:
- EP-A- 0 326 915
- WO-A-01/28474
- DE-A- 3 026 402
- GB-A- 2 059 963
- GB-A- 2 071 086
- GB-A- 2 093 027
- US-A- 4 045 576
- US-A- 4 126 635
- US-A- 4 254 146
- US-A- 4 313 949
- US-A- 4 503 073
- US-A- 4 568 695
- US-A- 4 683 242
- US-A- 5 475 034
- US-A- 5 811 446
- US-A- 6 066 671

## Description

### FIELD OF THE INVENTION

This invention relates to the use of certain 3-benzoylphenylacetic acids derivatives to treat or prevent angiogenic diseases, according to the present claims.

### BACKGROUND OF THE INVENTION

3-benzoylphenylacetic acid and certain of its derivatives are known to possess anti-inflammatory activity. U. S. Patent Nos. 4,254,146,4,045,576, 4,126,635, and 4,503,073, and U. K. Patent Application Nos. 2,071,086A and 2,093,027A disclose various 3-benzoylphenylacetic acids, salts and esters, and hydrates thereof, having anti-inflammatory activity. U. S. Patent No. 4,568,695 discloses 2-amino-3-benzoylphenylethyl alcohols having anti-inflammatory activity. U. S. Patent No. 4,313,949 discloses 2-amino-3-benzoylphenylacetamides having anti-inflammatory activity.

Certain derivatives of 2-amino-3-benzoylbenzeneacetic acid (amfenac) and 2-amino-3- (4-chloro-benzoyl) benzeneacetic acid have also been evaluated by Walsh et al., J. Med Chem., 33: 2296-2304 (1990), in an attempt to discover nonsteroidal anti-inflammatory prodrugs with minimal or no gastrointestinal side effects upon oral administration.

U. S. patent No. 4,683,242 teaches the transdermal administration of 2-amino-3-benzoylphenylacetic acids, salts, and esters, and hydrates and alcoholates thereof to control inflammation and alleviate pain.

U. S. Patent No. 4,910,225 teaches certain benzoylphenylacetic acids for local administration to control ophthalmic, nasal or otic inflammation. Only acetic acids are disclosed inthe'225 patent; no esters or amides are mentioned or taught as anti-inflammatory agents for local administration to the eyes, nose and ears.

U. S. Patent No. 5,475,034 discloses topically administrable compositions containing certain amide and ester derivatives of 3-benzyolphenylacetic acid, including nepafenac, useful for treating ophthalmic inflammatory disorders and ocular pain. According to the '034 patent at Col. 15, lines 35-39,"such disorders include, but are not limited to uveitis, scleritis, episcleritis, keratitis, surgically-induced inflammation and endophthalmitis.

U. S. Patent No. 6,066,671 discloses the topical use of certain amide and ester derivatives of 3-benzoylphenylacetic acid, including nepafenac, for treating GLC1A glaucoma.

WO 01/28474 A (Alcon) describes drug delivery devices, implantable adjacent the eye, for topical administration to the eye of compounds such as steroidal anti-inflammatory drugs, and nepafenac as a non-steroidal anti-inflammatory drug.

### SUMMARY OF THE INVENTION

It has now been found that certain 3-benzoylphenlacetic acids and derivatives, including nepafenac (2-amino-3-benzoyl-phenylacetamide), are useful for the manufacture of a medicament for treating disorders characterized, at least in part, by angiogenesis, in accordance with claims which follow.

### DETAILED DESCRIPTION OF THE INVENTION

The 3-benzoylphenylacetic acids derivatives useful in the present invention are those of formula (I) below. 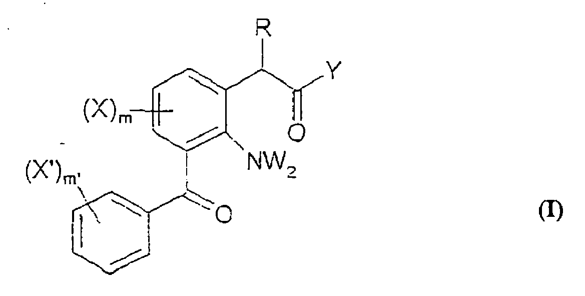 wherein
R = H, C₁₋₄ (un)branched alkyl, CF₃, SR⁴;
Y =OR',NR"R';
R' = H, C₁₋₁₀ (un)branched alkyl, (un)substituted (substitution as defined by X below), (un)substituted heterocycle (substitution as defined by X below), -(CH₂)ₙZ (CH₂)ₙA;
n = 2 - 6;
n' = 1 - 6;
Z = nothing, O, C=O, OC(=O), C(=O)O, C(=O)NR³, NR³C(=O), S(O)ₙ², CHOR³, NR³;
n²= 0 - 2;
R³ = H, C₁₋₆ (un)branched alkyl, (un)substituted aryl (substitution as defined by X below), (un)substituted heterocycle (substitution as defined by X below);
A = H, OH, optionally (un)substituted aryl (substitution as defined by X below), (un)substituted heterocycle (substitution as defined by X below), -(CH₂)ₙOR³;
R" = H, OH,OR';
X and X' independently = H, F, Cl, Br, I, OR', CN, OH, S(O)ₙ²R⁴, CF₃, R⁴, NO₂;
R⁴ = C ₁₋₆ (un)branched alkyl;
m =0-3;
m'=0-5;
W = O, H.

As used herein, the acid (Y = OH) includes pharmaceutical acceptable salts as well.

Preferred compounds for use according to the present invention are those of formula **(I)** wherein:
R = H, C₁₋₂akyl ;
Y = NR'R";
R' = H, C₁₋₆ (un)branched alkyl, -(CH²)_{*n*}Z(CH₂)_{*n*'}A;
Z = nothing, O, CHO_{R}³, NR³;
R³=H;
A = H, OH, (un)substituted aryl (substitution as defined by X below);
X and X' independently = H, F, Cl, Br, CN,CF₃, OR', SR⁴, R⁴;
R" = H;
R⁴ = C₁₋₄ (un)branched alkyl;
m = 0 - 2;
m' = 0-2;
W = H;
n = 2-4;
n' = 0-3.

The most preferred compounds for use in the present invention are 2-Amino-3-(4-fluorobenzoyl)-phenylacetamide; 2-Amino-3-benzoyl-phenylacetamide (nepafenac); and 2-Amino-3-(chlorobenzoyl)-phenylacetamide.

A therapeutically effective amount of a compound of formula **(I)** is to be administered topically, locally or systemically to treat or prevent angiogenesis-related disorders, namely those that involve the proliferation of tumor cells, selected from prostate cancer, lung cancer, breast cancer, bladder cancer, renal cancer, colon cancer, gastric cancer, pancreatic cancer, ovarian cancer, melanoma, hepatoma, sarcoma and lymphoma and the ophthalmic angiogenesis-related disorders exudative macular degeneration; proliferative diabetic retinopathy; ischemic retinopathy (e.g., retinal vein or artery occlusion); retinopathy of prematurity; neovascular glaucoma; iritis rubeosis; corneal neovascularization; and pterygium. Certain disorders, such as retinal vein or artery occlusion, can be characterized by both angiogenesis and neurodegenerative components. Accordingly, a compound of formula **(I)** is administered to treat or prevent disorders characterized, at least in part, by angiogenesis.

The compounds of formula ***(I)** can be administered in a variety of ways, including all forms of local delivery to the eye, such as subconjunctival injections or implants, intravitreal injections or implants, sub-Tenon's injections or implants, incorporation in surgical irrigating solutions, etc. Additionally, the compounds of formula **(I)** can be administered systemically, such as orally or intravenously. Suitable pharmaceutical vehicles or dosage forms for injectable compositions, implants, and systemic administration are known. The compounds of formula **(I)** and especially those wherein Y = NR'R", however, are preferably administered topically to the eye and can be formulated into a variety of topically administrable ophthalmic compositions, such as solutions, suspensions, gels or ointment.

Pharmaceutical compositions comprising a compound of formula **(I)** in aqueous solution or suspension, optionally containing a preservative for multidose use and other conventionally employed ophthalmic adjuvants, can be topically administered to the eye. The most preferred form of delivery is by aqueous eye drops, but gels or ointments can also be used. Aqueous eye drops, gels and ointments can be formulated according to conventional technology and would include one or more excipients. For example, topically administrable compositions may contain tonicity-adjusting agents, such as mannitol or sodium chloride; preservatives such as chlorobutanol, benzalkonium chloride, polyquaternium-1, or chlorhexidine; buffering agents, such as phosphates, borates, carbonates and citrates; and thickening agents, such as high molecular weight carboxy vinyl polymers, including those known as carbomers, hydroxyethylcellulose, or polyvinyl alcohol.

The doses of the compounds of formula **(I)** used in the treatment or prevention of ophthalmic angiogenesis-related disorders will depend on the type of disorder to be prevented or treated, the age and body weight of the patient, and the form of preparation/route of administration. Compositions intended for topical ophthalmic administration will typically contain a compound of formula **(I)** in an amount of from 0.001 to 4.0% (w/v), preferably from 0.01 to 0.5% (w/v), with 1-2 drops once to several times a day. Likewise, representative doses for other forms of preparations are approximately 1-100 mg/day/adult for injections and approximately 10-1000 mg/adult for oral preparations, each administered once to several times a day.

Additional therapeutic agents may be added to supplement the compounds of formula **(I).**

The following examples are presented to illustrate various aspects of the present invention. The percentages are expressed on a weight/volume basis.

Example 1: The following formulations are representative of the topical compositions useful in the present invention.

**Formulation 1**

| | |
|---|---|
| Compound of formula **(I)** | 0.01-0.5% |
| Polysorbate 80 | 0.01% |
| Benzalkonium Chloride | 0.01 % + 10% excess |
| Disodium EDTA | 0.1% |
| Monobasic Sodium Phosphate | 0.03% |
| Dibasic Sodium Phosphate | 0.1 % |
| Sodium Chloride | q. s. 290-300 mOsm/Kg |
| pH adjustment with NaOH and/or HCl | pH 4.2 - 7.4 |
| Water | q. s. 140% |

**Formulation 2**

| | |
|---|---|
| Compound of formula (I) | 0.01-0.5% |
| Hydroxypropyl Methylcellulose | 0.5% |
| Polysorbate 80 | 0.01 % |
| Benzalkonium Chloride | 0.01 % + 5% excess |
| Disodium EDTA | 0.01% |
| Dibasic Sodium Phosphate | 0.2% |
| Sodium Chloride | q. s. 290-300mOsm/Kg |
| pH adjustment withNaOH and/or HCl | pH 4.2 - 7.4 |
| Water | q. s. 100% |

**Formulation 3**

| | |
|---|---|
| Nepafenac | 0.1 + 6% excess |
| Carbopol 974P | 0.08% |
| Tyloxapol | 0.01 % |
| Glycerin | 2.4% |
| Disodium EDTA | 0.01% |
| Benzalkonium Chloride | 0.01% |
| pH adjustment with NaOH and/or HCl | pH 7.5 ± 0.2 |
| Water | q. s. 100% |

## Claims

1. Use of a 3-benzoylphenylacetic acid derivative having the following formula, in the manufacture of a medicament for treating or preventing angiogenesis related disorders in a patient suffering from or predisposed to such a disorder, selected from the group consisting of prostate cancer; lung cancer; breast cancer; bladder cancer; renal cancer; colon cancer; gastric cancer; pancreatic cancer; ovarian cancer; melanoma; hepatoma; sarcoma; and lymphoma: 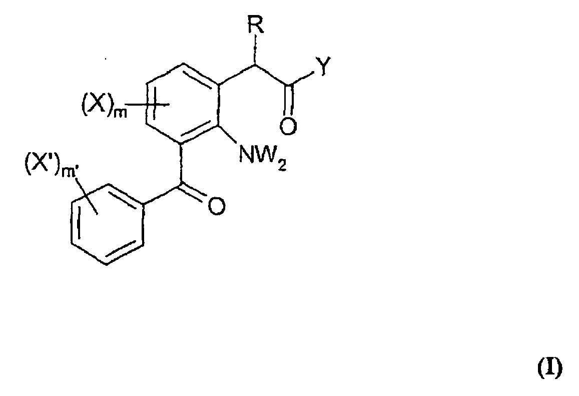 wherein
R = H, C₁₋₄ (un)branched alkyl, CF₃, SR⁴;
Y = OR', NR"R';
R' = H, C₁₋₁₀ (un)branched alkyl, (un)substituted (substitution as defined by X below), (un)substituted heterocycle (substitution as defined by X below), -(CH₂)_{*n*}Z (CH₂)_{*n*'}A;
n =2-6;
n' = 1 - 6;
Z = nothing, O, C=O, OC(=O), C(=O)O, C(=O)NR³, NR³C(=O), S(O)ₙ², CHOR³, NR³;
n² = 0 - 2;
R³ = H, C₁₋₆ (un)branched alkyl, (un)substituted aryl (substitution as defined by X below), (un)substituted heterocycle (substitution as defined by X below);
A = H, OH, optionally (un)substituted aryl (substitution as defined by X below), (un)substituted heterocycle (substitution as defined by X below), -(CH₂)_{*n*}OR³;
R" = H, OH,OR';
X and X' independently = H, F, Cl, Br, I, OR', CN, OH, S(O)ₙ²R⁴, CF₃, R⁴, NO₂;
R⁴ = C₁₋₆ (un)branched alkyl;
m =0-3;
m'=0-5;
W = O, H.

2. Use of a 3-benzoylphenylacetic acid derivative having the following formula, in the manufacture of a medicament for treating or preventing angiogenesis-related ophthalmic disorders in a patient suffering from or predisposed to such an ophthalmic disorder, selected from the group consisting of exudative macular degeneration; proliferative diabetic retinopathy; ischemic retinopathy; retinopathy of prematurity; neovascular glaucoma; iritis rubeosis; corneal neovascularization; and pterygium;. 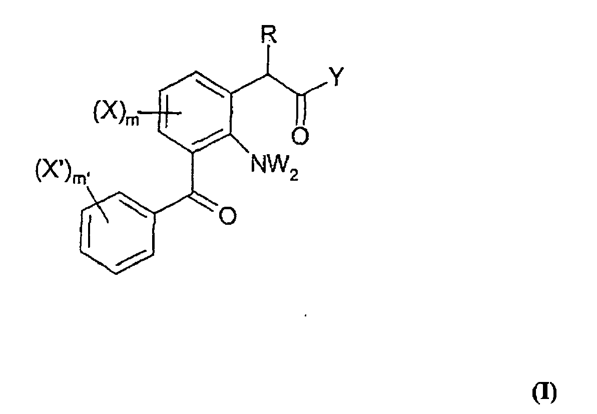 wherein
R = H, C₁₋₄ (un)branched alkyl, CF₃, SR⁴;
Y = OR', NR"R';
R' = H, C₁₋₁₀ (un)branched alkyl, (un)substituted (substitution as defined by X below), (un)substituted heterocycle (substitution as defined by X below), -(CH₂)ₙZ (CH₂)_{n'}A;
n =2-6;
n' = 1 - 6;
Z = nothing, O, C=O, OC(=O), C(=O)O, C(=O)NR³, NR³C(=O), S(O)ₙ², CHOR³, NR³;
n² = 0 - 2;
R³ = H, C₁₋₆ (un)branched alkyl, (un)substituted aryl (substitution as defined by X below), (un)substituted heterocycle (substitution as defined by X below);
A = H, OH, optionally (un)substituted aryl (substitution as defined by X below), (un)substituted heterocycle (substitution as defined by X below), -(CH₂)_{*n*}OR³;
R" = H, OH,OR';
X and X' independently = H, F, Cl, Br, I, OR', CN, OH, S(O)ₙ²R⁴, CF₃, R⁴, NO₂;
R⁴ = C₁₋₆ (un)branched alkyl;
m =0-3;
m'=0-5;
W = O, H.

3. Use, as in claim 1 or claim 2, wherein
R = H, C₁₋₂alkyl;
Y = NR'R";
R' = H, C₁₋₆ (un)branched alkyl, -(CH₂)_{*n*}Z(CH₂)_{*n*},A;
Z = nothing, O, CHOR³, NR³;
R³=H;
A = H, OH, (un)substituted aryl (substitution as defined by X below);
X and X' independently = H, F, Cl, Br, CN,CF₃, OR', SR⁴, R⁴;
R" = H;
R⁴ = C₁₋₄ (un)branched alkyl;
m = 0-2;
m'=0-2;
W = H;
n =2-4;
n'=0-3.

4. Use, as in claim 3, wherein the 3-benzoylphenylacetic acid derivative is selected from the group consisting of 2-Amino-3-(4-fluorobenzoyl)-phenylacetamide; 2-Amino-3-benzoyl-phenylacetamide; and 2-Amino-3-(4-chlorobenzoyl)-phenylacetamide.

5. Use, as in claim 4, wherein the 3-benzoylphenylacetic acid derivative is 2-Amino-3-benzoyl-phenyl acetamide (nepafenac).

6. Use, as in claim 2, wherein the medicament is to be topically administered to the eye.

7. Use, as in claim 6, wherein a therapeutically effective amount of 3-benzoylphenylacetic acid derivative is from 0.001 to 4.0% (w/v).

8. Use, as in claim 2, wherein the medicament is to be administered orally, intravenously, in a subconjunctival injection or implant, in a sub-Tenon's injection or implant, in an intravitreal injection or implant, or in a surgical irrigating solution.

## Patentansprüche

1. Verwendung eines 3-Benzoylphenylessigsäurederivats mit der folgenden Formel, zur Herstellung eines Medikaments zum Behandeln oder Verhindern von mit Angiogenese in Beziehung stehenden Erkrankungen in einem Patienten, der an einer Krankheit leidet oder für eine solche Krankheit prädisponiert ist, ausgewählt aus der Gruppe, bestehend aus Prostatakrebs, Lungenkrebs, Brustkrebs, Blasenkrebs, Nierenkrebs, Dickdarmkrebs, Magenkrebs, Bauchspeicheldrüsenkrebs, Ovarialkrebs, Melanom, Hepatom, Sarcom und Lymphom: 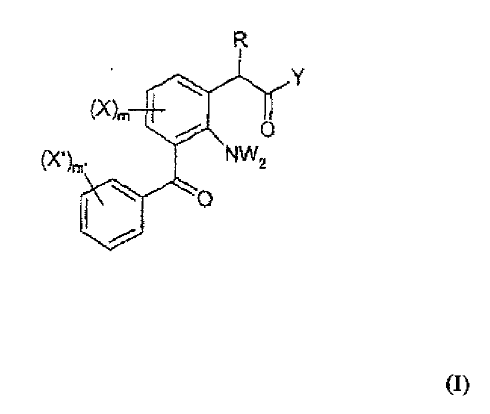 wobei
R = H, C₁₋₄ (un) verzweigtes Alkyl, CF₃, SR⁴;
Y = OR', NR"R';
R' = H, C₁₋₁₀ (un)verzweigtes Alkyl, (un)subsituiertes (Substitution wie unten durch X definiert), (un)substituierter Heterozyklus (Substitution wie unten durch X definiert), -(CH₂)ₙZ
(CH₂)ₙ,A;
n=2-6
n' = 1 - 6
Z = nichts, O, C = O, OC(= O), C(= O)O, O, C(=O)NR³, NR³C(=O), S(O)ₙ², CHOR³, NR³;
n²= 0 - 2;
R³ = H, C₁₋₆ (un)verzweigtes Alkyl, (un)substituiertes Aryl (Substitution wie unten durch X definiert), (un) substituierter Heterozyklus (Substitution wie unten durch X definiert);
A = H, OH, fakultativ (un)substituiertes Aryl (Substitution wie unten durch X definiert), (un)substituierter Heterozyklus (Substitution wie unten durch X definiert), -(CH₂)ₙOR³;
R" = H, OH; OR';
X und X' unabhängig = H, F, Cl, Br, I, OR', CN, OH, S(O)ₙ²R⁴, CF₃, R⁴, NO₂;
R⁴ = C₁₋₆ (un)verzweigtes Alkyl;
m = 0 - 3;
m' = 0 - 5;
W = O,H.

2. Verwendung eines 3-Benzoylphenylessigsäurederivats mit der folgenden Formel, zur Herstellung eines Medikaments zum Behandeln oder Verhindern von mit Angiogenese in Beziehung stehenden Augenerkrankungen in einem Patienten, der an einer solchen Augenerkrankung leidet oder für eine solche Augenerkrankung prädisponiert ist, ausgewählt aus der Gruppe, bestehend aus exsudativer Makula-Degeneration, proliferativer Diabetes-Retinopathie, ischämischer Retinopathie, Frühgeborenen-Retinopathie, neovaskuläres Glaukom, Iritis rubeosis; Kornea-Neovaskularisation und Pterygium: 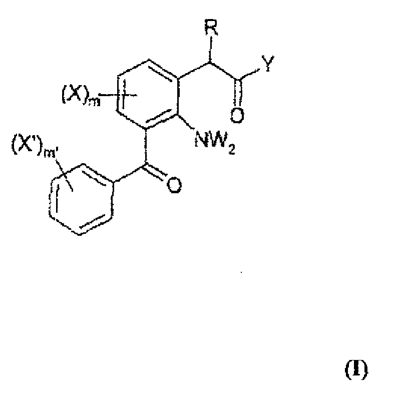 wobei
R = H, C₁₋₄ (un)verzweigtes Alkyl, CF₃, SR⁴;
Y = OR', NR"R';
R' = H, C₁₋₁₀ (un)verzweigtes Alkyl, (un)substituiertes (Substitution wie unten durch X definiert), (un)substituierter Heterozyklyus (Substitution wie unten durch X definiert), -(CH₂)ₙZ
(CH₂)_{n'}A;
n=2-6;
n' = 1-b;
Z = nichts, O, C = O, OC(=O), C(=O)O, C(=O)NR³, NR³C(=O), S(O)ₙ², CHOR³, NR³;
n² = 0 - 2;
R³ = H, C₁₋₆ (un)verzweigtes Alkyl, (un)substituiertes Aryl (Substitution wie unten durch X definiert), (un)substituierter Heterozyklus (Substitution wie unten durch X definiert);
A = H, OH, fakultativ (un)substituiertes Aryl (Substitution wie unten durch X definiert), (un)substituierter Heterozyklus (Substitution wie unten durch X definiert), -(CH₂)ₙOR³;
R" = H, OH, OR';
X und X' unabhängig = H, F, Cl, Br, I, OR', CN, OH, S(O)ₙ²R⁴, CF₃, R⁴, NO₂;
R⁴ = C₁₋₆ (un)verzweigtes Alkyl;
m = 0 - 3;
m' = 0 - 5;
W = O, H.

3. Verwendung nach Anspruch 1 oder Anspruch 2, wobei
R = H, C₁₋₂-Alkyl;
Y = NR'R";
R' = H, C₁₋₆(un)verzweigtes Alkyl, -(CH₂)ₙZ(CH₂)ₙA;
Z = nichts, O, CHOR³, NR³;
R³ = H;
A = H, OH, (un)substituiertes Aryl (Substitution wie unten durch X definiert);
X und X' unabhängig = H, F, Cl, Br, CN, CF₃, OR', SR⁴, R⁴;
R" = H;
R⁴ = C₁₋₄ (un)verzweigtes Alkyl;
m = 0 - 2;
m' = 0 - 2;
W = H;
n = 2 - 4;
n' = 0 - 3.

4. Verwendung nach Anspruch 3, wobei das 3-Benzoylphenylessigsäurederivat ausgewählt ist aus der Gruppe, bestehend aus 2-Amino-3-(4-fluorobenzoyl)-phenylacetamid; 2-Amino-3-benzoyl-phenylacetamid; und 2-Amino-3-(4-chlorbenzoyl)-phenylacetamid ausgewählt ist.

5. Verwendung nach Anspruch 4, wobei das 3-Benzoylphenylessigsäurederivat 2-Amino-3-benzoyl-phenylacetamid (nepafenac) ist.

6. Verwendung nach Anspruch 2, wobei das Medikament topisch an das Auge verabreicht werden soll.

7. Verwendung nach Anspruch 6, wobei eine therapeutisch wirksame Menge an 3-Benzoylphenylessigsäurederivat im Bereich von 0,001 bis 4,0 % (Gew./Vol.) liegt.

8. Verwendung nach Anspruch 2, wobei das Medikament oral, intravenös, in einer subkonjunktivalen Injektion oder Implantat, in einer Sub-Tenon-Injektion oder Implantat ("sub-Tenon's injection"), in einer Intravitrealinjektion oder Implantat, oder in einer Operationsspüllösung verabreicht werden soll.

## Revendications

1. Utilisation d'un dérivé d'acide 3-benzoylphénylacétique ayant la formule suivante, pour la fabrication d'un médicament destiné à traiter ou prévenir des troubles associés à une angiogenèse chez un patient souffrant d'un tel trouble, ou prédisposé à un tel trouble, sélectionné parmi le groupe constitué du cancer de la prostate, cancer du poumon, cancer du sein, cancer de la vessie, cancer du rein, cancer du colon, cancer gastrique, cancer pancréatique, cancer ovarien, mélanome, hépatome, sarcome, et lymphome : 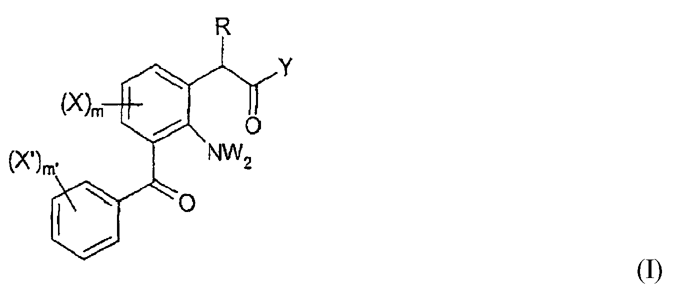 où
R = H, un alkyle (non)ramifié C₁₋₄, CF₃, SR⁴,
Y = OR', NR"R',
R' = H, un alkyle (non)ramifié C₁₋₁₀, (non)substitué (substitution comme défini par X ci-dessous), un hétérocycle (non)substitué (substitution comme défini par X ci-dessous), -(CH₂)ₙZ(CH₂)_{n'}A,
n = 2-6,
n' = 1-6,
Z = rien, O, C=O, OC(=O), C(=O)O, C(=O)NR³, NR³C(=O), S(O)ₙ², CHOR³, NR³,
n² = 0-2:
R³ = H, un alkyle (non)ramifié C₁₋₆, un aryle (non)substitué (substitution comme défini par X ci-dessous), un hétérocycle (non)substitué (substitution comme défini par X ci-dessous),
A = H, OH, de manière facultative un aryle (non)substitué (substitution comme défini par X ci-dessous), un hétérocycle (non)substitué (substitution comme défini par X ci-dessous), ―(CH₂)ₙOR³,
R" = H, OH, OR',
X et X' indépendamment = H, F, Cl, Br, I, OR', CN, OH, S(O)ₙ² R⁴, CF₃, R⁴, NO₂,
R⁴ = un alkyle (non)ramifié C₁₋₆,
m = 0-3,
m' = 0-5,
W = O, H.

2. Utilisation d'un dérivé d'acide 3-benzoylphénylacétique ayant la formule suivante, pour la fabrication d'un médicament destiné à traiter ou prévenir des troubles ophtalmiques associés à une angiogenèse chez un patient souffrant d'un tel trouble ophtalmique ou prédisposé à un tel trouble, sélectionné parmi le groupe constitué d'une dégénérescence maculaire exsudative, rétinopathie diabétique proliférative, rétinopathie ischémique, rétinopathie des prématurités, glaucome néo-vasculaire, rubéose de l'iris, néovascularisation cornéenne, et ptérygion, 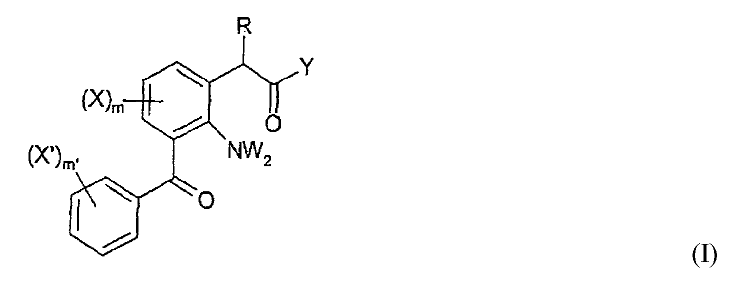 où
R = H, un alkyle (non)ramifié C₁₋₄, CF₃, SR⁴,
Y= OR', NR"R',
R' = H, un alkyle (non)ramifié C₁₋₁₀, (non)substitué (substitution comme défini par X ci-dessous), un hétérocycle (non)substitué (substitution comme défini par X ci-dessous), -(CH₂)ₙZ(CH₂)_{n'}A,
n = 2-6,
n' = 1-6,
Z = rien, O, C=O, OC(=O), C(=O)O, C(=O)NR³, NR³C(=O), S(O)ₙ², CHOR³, NR³,
n² = 0-2:
R³ = H, un alkyle (non)ramifié C₁₋₆, un aryle (non)substitué (substitution comme défini par X ci-dessous), un hétérocycle (non)substitué (substitution comme défini par X ci-dessous),
A = H, OH, de manière facultative un aryle (non)substitué (substitution comme défini par X ci-dessous), un hétérocycle (non)substitué (substitution comme défini par X ci-dessous), ―(CH₂)ₙOR³,
R" = H, OH, OR',
X et X' indépendamment = H, F, Cl, Br, I, OR', CN, OH, S(O)ₙ² R⁴, CF₃, R⁴, NO₂,
R⁴ = un alkyle (non)ramifié C₁₋₆,
m = 0-3,
m' = 0-5,
W = O, H.

3. Utilisation selon la revendication 1 ou la revendication 2, dans laquelle
R = H, un alkyle C₁₋₂,
Y = NR'R",
R' = H, un alkyle (non)ramifié C₁₋₆, -(CH₂)ₙZ(CH₂)ₙA,
Z = rien, O, CHOR³, NR³,
R³ = H,
A = H, OH, un aryle (non)substitué (substitution comme défini par X ci-dessous),
X et X' indépendamment = H, F, Cl, Br, CN, CF₃, OR', SR⁴, R⁴,
R" = H,
R⁴ = un alkyle (non)ramifié C₁₋₄,
m = 0-2,
m' = 0-2,
W = H,
n = 2-4,
n' = 0-3.

4. Utilisation selon la revendication 3, dans laquelle le dérivé d'acide 3-benzoylphénylacétique est sélectionné parmi le groupe constitué de 2-Amino-3-(4-fluorobenzoyl)-phénylacétamide, 2-Amino-3-benzoyl-phénylacétamide, et 2-Amino-3-(4-chlorobenzoyl)-phénylacétamide.

5. Utilisation selon la revendication 4, dans laquelle le dérivé d'acide 3-benzoylphénylacétique est le 2-Amino-3-benzoyl-phénylacétamide (nepafenac).

6. Utilisation selon la revendication 2, dans laquelle le médicament est à administrer topiquement dans l'oeil.

7. Utilisation selon la revendication 6, dans laquelle une quantité thérapeutiquement efficace du dérivé d'acide 3-benzoylphénylacétique est comprise entre 0,001 et 4,0 % (p/v).

8. Utilisation selon la revendication 2, dans laquelle le médicament est à administrer oralement, par voie intraveineuse, dans une injection ou un implant sous-conjonctival, dans une injection ou un implant sous-Tenon, dans une injection ou un implant intravitréal, ou dans une solution d'irrigation chirurgicale.
